# EUROPEAN PATENT SPECIFICATION

(11) **EP 0 398 578 B1**
(45) Date of publication and mention of the grant of the patent: **12.03.1997**
(21) Application number: 90304975.7
(22) Date of filing: 09.05.1990
(51) Int. Cl.: C07D 451/02, A61K 31/33, A61K 31/695, C07D 211/54, C07D 211/48, C07D 491/10, C07D 451/06, C07D 207/12, C07F 7/18

(54) **2-piperidino-1-alkanol derivatives as antiischemic agents**
2-Piperidino-1-alkanol-Derivate als antiischemische Wirkstoffe
2-Pipéridino-1-alkanol dérivés comme agents anti-ischémiques

(30) Priority: 17.05.1989 WO PCT/US89/02176; 16.01.1990 WO PCT/US90/00292
(43) Date of publication of application: 22.11.1990
(73) Proprietor: PFIZER INC., New York, N.Y. 10017 (US)
(72) Inventor: Chenard, Bertrand Leo, Waterford, Connecticut (US)
(74) Representative: Moore, James William, Dr.

(56) References cited:
- EP-A- 0 053 744
- EP-A- 0 076 089
- US-A- 4 366 154
- CHEMICAL ABSTRACTS, vol. 105, no. 5, 4th August 1986, page 725, abstract no. 42659p, Columbus, Ohio, US; & JP-A-61 36 262

## Description

The present invention is directed to neuroprotective (antiischemic and excitory aminoacid receptor blocking) 2-piperidino-1-alkanol derivatives defined by the formula (11) below and pharmaceutically acceptable salts thereof; for use in the treatment of stroke or CNS degenerative diseases such as Alzheimer's disease, Huntington's disease and Parkinson's disease; and to certain intermediates therefor.

Ifenprodil is a racemic, so-called dl-erythro compound having the relative stereochemical formula 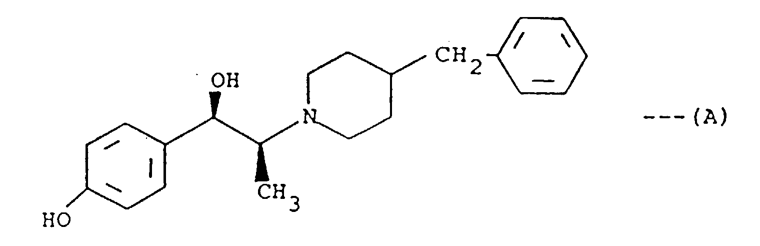 which is marketed as a hypotensive agent, a utility shared by a number of close analogs; Carron et al., U.S. Patent 3,509,164; Carron et al., Drug Res., v. 21, pp. 1992-1999 (1971). More recently, ifenprodil has been shown to possess antiischemic and excitory aminoacid receptor blocking activity; Gotti et al., J. Pharm. Exp. Therap., v. 247, pp. 1211-21 (1988); Carter et al., loc. cit., pp. 1222-32 (1988). See also French Patent 2546166. A goal, substantially met by the present invention, has been to find compounds possessing such neuroprotective effect in good measure, while at the same time having lowered or no significant hypotensive effect.

Certain structurally related 1-phenyl-3-(4-aryl-4-acyloxypiperidino)-1-propanols have also been reported to be useful as analgesics, U.S. Patent 3,294,804; and 1-[4-(amino- and hydroxy-alkyl)phenyl]-2-(4-hydroxy-4-tolylpiperazino)-1-alkanols and alkanones have been reported to possess analgesic, antihypertensive, psychotropic or antiinflammatory activity, Japanese Kokai 53-02,474 (CA 89:43498y; Derwent Abs. 14858A) and 53-59,675 (CA 89:146938w; Derwent Abs. 48671A).

Structurally related nortropanol derivatives are disclosed in EP-A-0053744 and in U.S. Patent no 4366154. The compounds are neuroleptic agents of utility as tranquilizers. Chemical Abstracts 1986, 105,725 (abstract no 42659p) discloses tetrahydropyridinylpropanones and -propanols as intermediates for the preparation of ifenprodil.

The present invention is directed to compounds of the formula : 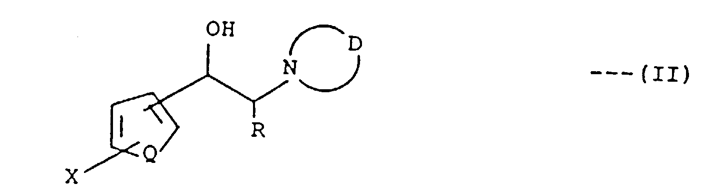 wherein
D is 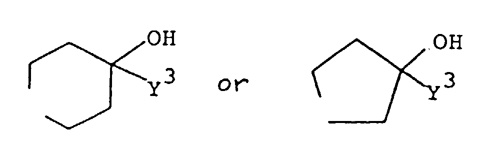
R is H, (C₁-C₆)alkyl, (C₂-C₆)alkenyl or (C₂-C₆)alkynyl;
X is hydrogen, (C₁-C₃)alkyl, halo, OR¹, OCOR¹, CO₂R¹, SR¹, NHR¹, NHCOR¹, CONH₂, or CN;
R¹ is hydrogen or (C₁-C₃)alkyl;
Q is S or CH = CH;
and Y³ is 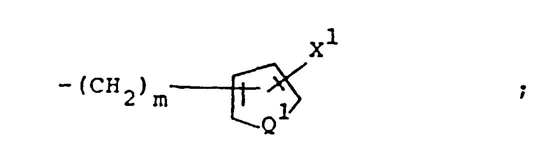
   wherein Q¹ is independently a value of Q as defined above;
   X¹ is independently a value of X as defined above and
   m is 0, 1, 2, 3 or 4;
   and pharmaceutically acceptable acid addition salts thereof.

The expression "pharmaceutically acceptable acid addition salts" is intended to include but is not limited to such salts as the hydrochloride, hydrobromide, hydroiodide, nitrate, hydrogen sulfate, dihydrogen phosphate, mesylate, maleate, and succinate. Such salts are conventionally prepared by reacting the free base form of the compound (II) with an appropriate acid, usually one molar equivalent, and in a solvent. Those salts which do not precipitate directly are generally isolated by concentration of the solvent and/or addition of a non-solvent.

The preferred compounds of the present invention generally have R as methyl and possess 1S*, 2S* or threo relative stereochemistry at the 1- and 2-positions of the propanol chain, i.e., 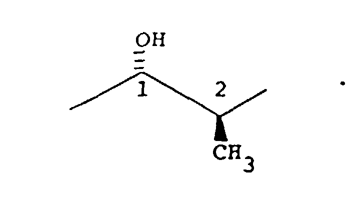 Particular individual compounds include (+)-(1S,2S)-1-(4-hydroxyphenyl)-2-(4-hydroxy-4-phenyl-piperidinyl)-1-propanol and (-)-(1R,2R)-1-(4-hydroxyphenyl)-2-(4-hydroxy-4-phenylpiperidinyl)-1-propanol.

The present invention is also directed to pharmaceutical compositions for treating a mammal, particularly man, suffering a central nervous disorder, which comprise a neuroprotective effective amount of a compound of the formula. Said compositions are particularly valuable in the treatment of stroke, Alzheimer's disease, Parkinson's disease, Huntington's disease and related disorders of the central nervous system.

The present invention is further directed to intermediate compounds of the formula 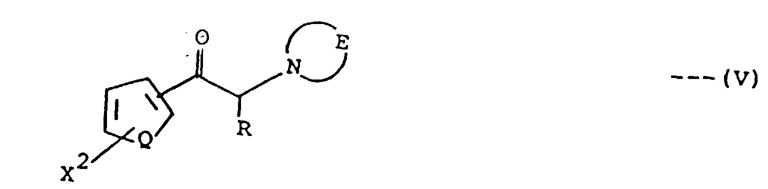 wherein
R is hydrogen, (C₁-C₆)alkyl, (C₂-C₆)alkenyl or (C₂-C₆)alkynyl;
Q is S or CH = CH;
X² is hydrogen, (C₁-C₃)alkyl, halo, OR¹, OR², COOR¹, OCOR¹, SR¹, SR², NHR¹, NHR¹R³, NHCOR¹, CONH₂ or CN;
R¹ is hydrogen or (C₂-C₃)alkyl;
R² is a conventional hydroxy or mercaptan protecting group;
R³ is a conventional amino protecting group;
E is 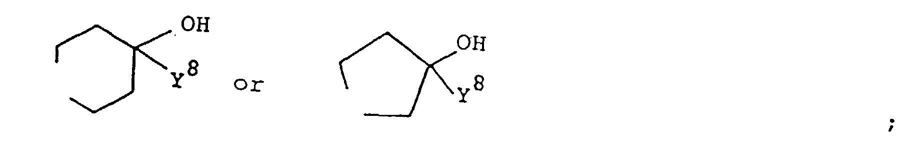
Y⁸ is 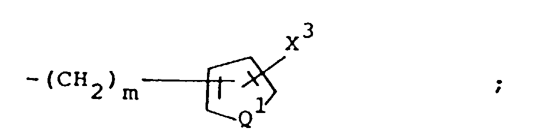
wherein
Q¹ is independently a value of Q as defined above;
X³ is independently a value of X² as defined above and m is O, 1, 2, 3 or 4.

It will be noted that those compounds of the invention which are 1-alkanols possess an asymmetric C-1 carbon, while those wherein R is other than hydrogen possess a second asymmetric center at the C-2 carbon of the alkanol. Similarly, in those compounds of the formula (V) which are 1-alkanones wherein R is other than hydrogen possess a C-2 asymmetric carbon. It will be evident to those skilled in the art of organic chemistry, therefore, that such compounds can be resolved into optical isomers showing equal but opposite rotation of plane polarized light. For example, all of these compounds are potentially resolved by fractional crystallization of their diastereomeric addition salts with an optically active acid, as exemplified below; while the alcohols are also potentially resolved by chromatography or fractional crystallization of esters derived by reaction with activated forms of optically active acids or with optically active isocyanates. Alternatively, optically active forms of certain of the present compounds are obtained by reaction of an appropriate amine with an optically active epoxide, as also exemplified below. Thus, the present invention should not be construed as limited to the racemic forms of the present compounds.

The compounds of the present invention, having the formula (II) defined above, are readily and generally prepared by nucleophilic displacement followed by reduction of the resulting ketone to alcohol as detailed below.

The precursor ketones are generally initially prepared with -OH, -SH and -NHR¹ groups in protected form, i.e., as -OR², -SR² or -NR¹R³ groups in the compounds of the formula (V).

Such protected ketones are generally formed by nucleophilic displacement of an appropriately substituted 2-halo, 2-alkanesulfonyloxy- or 2-arylsulfonyloxy-1-alkanone with an appropriately substituted piperidine derivative, e.g., 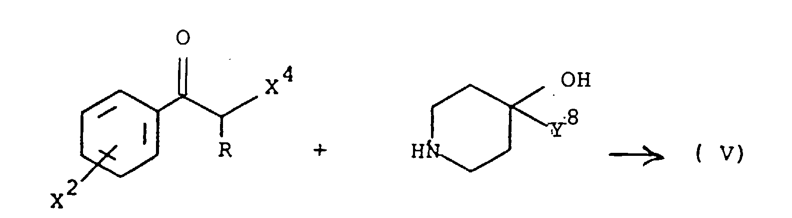 wherein X⁴ is typically chloro, bromo, mesyloxy or tosyloxy. This reaction is carried out under conditions typical of nucleophilic displacements in general. Where the two reactants are about equivalent in availability, close to substantially molar equivalents may be used; although when one is more readily available, it is usually preferred to use that one in excess, in order to force this bimolecular reaction to completion in a shorter period of time. The reaction is generally carried out in the presence of at least 1 molar equivalent of a base, the piperidine derivative itself, if it is readily available, but more usually a tertiary amine which is at least comparable in base strength to the nucleophilic piperidine; and in a reaction inert solvent such as ethanol. If desired, the reaction is catalyzed by the addition of up to one molar equivalent or more of an iodide salt (e.g., NaI, KI). Temperature is not critical, but will generally be somewhat elevated in order to force the reaction to completion within a shorter time period, but not so high as to lead to undue decomposition. A temperature in the range of 50-120°C is generally satisfactory. Conveniently, the temperature is the reflux temperature of the reaction mixture.

As used in the preceding paragraph, and elsewhere herein, the expression "reaction inert solvent" refers to any solvent which does not interact with starting materials, reagents, intermediates or products in a manner which adversely affects the yield of the desired product.

If desired, those ketone intermediates having OH, SH or NHR¹ groups in protected form (OR², SR² or NR¹R³) can be deprotected at this stage by conventional methods. For example, when R² is triisopropylsilyl or tert-butyldimethylsilyl, the protecting group is conveniently removed by reaction with tetrabutylammonium fluoride (generally, substantially 2 molar equivalents) in a reaction inert solvent such as tetrahydrofuran. When R² is benzyl or R³ is benzyloxycarbonyl, the protecting group will generally be removed by conventional hydrogenolysis over a noble metal catalyst in a reaction inert solvent, e.g., using 10% Pd/C as catalyst, preferably at low pressures (e.g., 1-10 atmospheres) and temperatures (e.g., 20-75°C) and generally in a reaction inert solvent such as methanol.

Generally excluding ketone intermediates containing ester groups or protecting groups such as benzyloxycarbonyl (which will generally be removed prior to ketone reduction), but otherwise with or without prior removal of protecting groups, the ketone intermediates are conveniently converted to corresponding alcohols by conventional reduction with a LiAlH₄, usually in excess (e.g., mol for mol), in a reaction inert solvent such as tetrahydrofuran at reduced temperature (e.g., -15°C to 15°C). Alternatively, ketone intermediates, particularly those containing ester groups, are reduced with a milder hydride reducing agent such as NaBH₄, again usually in excess, now in a protic solvent such as methanol or ethanol, generally at somewhat higher temperature, e.g., 15-45°C.

Any protecting groups which are still in place after ketone reduction are then removed according to the methods described above. Certain other transformations, such as olefin hydrogenation, epoxidation and sodium/liquid NH₃ reduction of epoxides, e.g., 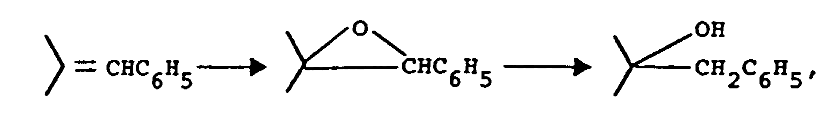 are also optionally carried out late in the synthetic sequence, e.g., after coupling to form ketone, after removal of protecting groups (so long as the unprotected groups do not interfere with the transformations) and/or after reduction of ketone to alcohol.

Said epoxidations are readily accomplished, for example, by reacting a methylene compound with substantially one molar equivalent of m-chloroperbenzoic acid in a reaction inert solvent such as CH₂Cl₂. The reduction of epoxide to alcohol is readily accomplished by conventional sodium/liquid ammonia, generally carried out at temperatures below the boiling point of liquid NH₃ (e.g., at -78°C, the temperature of an acetone-dry ice bath) in the presence of a reaction inert solvent such as tetrahydrofuran.

The starting materials and reagents required for the synthesis of the compounds of the present invention are readily available, either commercially, according to literature methods, or by methods exemplified in Preparations below.

The present compounds of the formula (II) possess selective neuroprotective activity, based upon their antiischemic activity and ability to block excitory aminoacid receptors, while at the same time generally having lowered or no significant hypotensive activity. The antiischemic activity of the present compounds is determined according to one or more of the methods which have been detailed previously by Gotti et al. and Carter et al. cited above, or by similar methods. The ability of the compounds of the present invention to block excitatory amino acid receptors is demonstrated by their ability to block N-methyl-D-aspartic acid-induced (NMDA) elevations of cGMP in neonatal rat cerebellums according to the following procedure. Cerebellums from ten 8-14 day old Wistar rats are quickly excised and placed in 4° C. Krebs/bicarbonate buffer, pH 7.4 and then chopped in 0.5 mm x 0.5 mm sections using a McIlvain tissue chopper (The Nickle Laboratory Engineering Co., Gomshall, Surrey, England). The resulting pieces of cerebellum are transferred to 100 ml of Krebs/ bicarbonate buffer at 37° C. which is continuously equilibrated with 95:5 O₂/CO₂. The pieces of cerebellum are incubated in such a manner for ninety minutes with three changes of the buffer. The buffer then is decanted, the tissue centrifuged (1 min., 3200 r.p.m.) and the tissue resuspended in 20 ml of the Krebs/bicarbonate buffer. Then, 250 µl aliquots (approximately 2 mg) are removed and placed in 1.5 ml microfuge tubes. To those tubes are added 10 µl of the compound under study from a stock solution followed, after a 10 minute incubation period, by 10 µl of a 2.5 mM solution of NMDA to start the reaction. The final NMDA concentration is 100 µM. Controls do not have NMDA added. The tubes are incubated for one minute at 37° C. in a shaking water bath and then 750 µl of a 50 mM Tris-Cl, 5mM EDTA solution is added to stop the reaction. The tubes are placed immediately in a boiling water bath for five minutes. The contents of each tube then are sonicated for 15 seconds using a probe sonicator set at power level three. Ten microliters are removed and the protein determined by the method of Lowry, Anal. Biochem. 100:201-220 (1979). The tubes are then centrifuged (5 min., 10,000 xg), 100 µl of the supernatant is removed and the level of cyclic GMP (cGMP) is assayed using a New England Nuclear (Boston, Massachusetts) cGMP RIA assay according to the method of the supplier. The data is reported as pmole cGMP generated per mg. protein. Undesired hypotensive activity is also determined by known methods, for example, according to the methods of Carron et al., also cited above.

Such selective neuroprotective antiischemic and excitatory amino acid blocking activities reflect the valuable utility of the present compounds in the treatment of degenerative CNS (central nervous system) disorders such as stroke; and Alzheimer's disease, Parkinson's disease and Huntington's disease; without significant potential for concurrent undue drop in blood pressure. In the systemic treatment of such diseases with a neuroprotective amount of compounds of the formula (II) the dosage is typically from about 0.02 to 10 mg/kg/day (1-500 mg/day in a typical human weighing 50 kg) in single or divided doses, regardless of the route of administration. Of course, depending upon the exact compound and the exact nature of the individual illness, doses outside this range may be prescribed by the attending physician. The oral route of administration is generally preferred. However, if the patient is unable to swallow, or oral absorption is otherwise impaired, the preferred route of administration will be parenteral (i.m., i.v.) or topical.

The compounds of the present invention are generally administered in the form of pharmaceutical compositions comprising at least one of the compounds of the formula (II) together with a pharmaceutically acceptable vehicle or diluent. Such compositions are generally formulated in a conventional manner utilizing solid or liquid vehicles or diluents as appropriate to the mode of desired administration: for oral administration, in the form of tablets, hard or soft gelatin capsules, suspensions, granules, powders and the like; for parenteral administration, in the form of injectable solutions or suspensions, and the like; and for topical administration, in the form of solutions, lotions, ointments, salves and the like.

The present invention is illustrated by the following examples, but is not limited to the details thereof.

All non-aqueous reactions were run under nitrogen for convenience and generally to maximize yields. All solvents/diluents were dried according to standard published procedures or purchased in a predried form. All reactions were stirred either magnetically or mechanically. NMR spectra are recorded at 300 MHz and are reported in ppm. The NMR solvent was CDCl₃ unless otherwise specified. IR spectra are reported in cm⁻¹, generally specifying only strong signals.

### ILLUSTRATIVE EXAMPLE A

### 2-(3-Phenylmethylene-8-azabicyclo[3.[3.2.1]oct-8-yl)-1-(4-(triisopropylsilyloxy)phenyl)-1-propanone

A mixture of 3-phenylmethylene-8-azabicyclo-[3.2.1]octane (1.09 g, 4.87 mmol), 4-(triisopropylsiloxy)-alpha-bromopropiophenone (1.88 g, 4.88 mmol) and triethylamine (1.5 ml, 10.76 mmol) in ethanol (75 ml) was refluxed 22 hours. After cooling, ether (50 ml) was added and the mixture was filtered through diatomaceous earth. The filtrate was concentrated and chromatographed on silica gel (2 x 6 inches, hexane then ethyl acetate/hexane gradient) to give 0.56 g (23%) of an orange oil product; NMR 8.18 (d, 2H), 7.25 (t, 2H), 7.12 (d, 3H), 6.86 (d, 2H), 6.29 (s, 1H), 4.08 (m, 1H), 3.47-3.26 (m, 2H), 2.75 (m, 1H), 2.57-2.37 (m, 2H), 2.03-1.72 (m, 4H), 1.6 (m, 1H; partially under water peak from NMR solvent), 1.40 (d, 3H), 1.25 (m, 3H), 1.09 (d, 18H).

### ILLUSTRATIVE EXAMPLE B

### Mixture of (1R*,2S*)- and (1S*,2S*)-2-(3-phenylmethylene-8-azabicyclo[3.2,1]oct-8-yl)-1-(4-(triisopropylsilyloxy)phenyl)-1-propanol

To a slurry of LiAlH₄ (0.61 g, 16.07 mmol) in tetrahydrofuran (50 ml) at 0°C was added title product of the preceding Example (8.04 g, 15.96 mmol) in tetrahydrofuran (150 ml) over 15 minutes. The mixture was stirred 15.5 hours at room temperature, then carefully quenched with water (1.2 ml), filtered over diatomaceous earth and concentrated to give a yellow oil (7.15 g, 89%). This mixture of racemic title products was used directly in the next reaction without purification.

### ILLUSTRATIVE EXAMPLE C

### (1S*,2S*)- and (1R*,2S*)-1-(4-hydroxyphenyl)-2-(3-phenylmethylene-8-azabicyclo[3.2.1]oct-8-yl)-1-propanol

Title product of the preceding Example (7.15 g, 14.14 mmol) was dissolved in tetrahydrofuran (250 ml) and tetrabutylammonium fluoride (28.5 ml, 28.5 mmol, 1M in tetrahydrofuran) was added all at once. The solution was stirred at room temperature 18 hours, then concentrated and chromatographed on silica gel (4 x 6 inches, ethyl acetate/hexane gradient followed by methanol/ethyl acetate gradient) to give first the racemic (1S*,2S*)-title product (1.58 g) followed by the more polar racemic (1R*,2S*)-title product (2.88 g). (Note that because of the asymmetry in the 3-phenylmethylene-8-azabicyclo[3.2.1]oct-8-yl side chain, each of these products is actually a mixture of two racemates).

The (1S*,2S*)-product was recrystallized from ethyl acetate/hexane to give 0.923 g of white solid; mp 175-177°C; NMR includes 4.10 (t, J=7.7Hz, 1H); Anal. C 78.77, H 7.90, N 3.92, calcd. C 79.05, H 7.90, N 3.92.

The (1R*,2S*)-product was further purified by radial chromatography with 60% ethyl acetate/hexane elution to give 0.24 g of colorless oil. This oil was crystallized from ether/hexane to give 0.17 g of fluffy solid, mp 78.5-85°C. The latter was converted to its HCl -salt by bubbling HCl gas into an ether solution of the compound for 3 minutes. The white precipitate was collected and recrystallized from ethanol to yield the (1R*,2S*)-hydrochloride salt as its half hydrate; mp 215-218°C; NMR (DMSO-d₆) includes 5.17 (s, 1H) and 4.60-3.93 (m, 2H); Anal. C 70.00, H 7.45, N 3.35, calcd. for ½ H₂O C 69.95, H 7.40, N 3.54.

### EXAMPLE 1

### 1-(4-(Benzyloxy)phenyl)-2-(4-benzyl-4-hydroxypiperidino)-1-propanone

This product was prepared following the procedure of Example A from 4-hydroxy-4-hydroxy-4-benzylpiperidine (2.0 g, 10.46 mmol), triethylamine (1.46 ml, 10.47 mmol), and 4-benzyloxy-alpha-bromopropiophenone (3.33 g, 10.43 mmol) in ethanol (50 ml) with a reflux period of 24 hours. The present racemic product was obtained after silica gel flash chromatography with ethyl acetate/hexane gradient elution. The yield was 2.88 g (64%) of a yellow solid; NMR 8.06 (d, 2H), 7.52-7.08 (m, 10H), 6.97 (d, 2H), 5.11 (s, 2H), 4.00 (q, 1H), 2.72 (s, 2H), 2.72-2.53 (m, 2H), 2.43 (t, 1H), 1.85-1.39 (m, 6H), 1.23 (d, 3H). HRMS 412.2348, calcd. (-OH) 412.2273.

### EXAMPLE 1

### (1S*,2S*)-1-(4-(Benzyloxy)phenyl)-2-(4-benzyl-4-hydroxypiperidino)-1-propanol

NaBH₄ (0.25 g, 6.61 mmol) was added all at once to a solution of title product of the preceding Example (2.88 g, 6.70 mmol) in ethanol (50 ml). The mixture was stirred 20 hours at ambient temperature as a precipitate formed. The solid was filtered and dried to yield 0.60 g of present title product; mp 147-148°C; NMR includes 4.17 (d, J = 10Hz, 1H); IR (KBr) 3387, 3024, 2936, 2909, 1611, 1513, 1453, 1239, 1026, 1011, 695. The filtrate from the above reaction was concentrated, the residue partitioned between ethyl acetate and water and the phases separated. The aqueous phase was extracted with ethyl acetate. The combined organic phases were washed with water, dried (CaSO₄) and concentrated to give 2.82 g of additional title product.

### EXAMPLE 3

### (1S*,2S*)-1-(4-Hydroxyphenyl)-2 -(4-benzyl-4-hydroxypiperidino)-1-propanol

Title product of the preceding Example (0.49 g, 1.14 mmol) and tetrahydrofuran (30 ml) was chilled to -78°C and ammonia gas (30 ml) was condensed into the mixture. Sodium (0.082 g, 3.57 mmol) was added in four pieces. The reaction, which gradually turned blue, was stirred 15 minutes and then quenched with ammonium chloride (0.29 g). The reaction was allowed to warm to ambient temperature, with the ammonia boiling off. The reaction was concentrated and the residue was taken up in ethyl acetate and washed with water and brine. The organic phase was dried (CaSO₄) and concentrated to give 0.39 g of white solid. Recrystallization from hexane gave 0.19 g of present title product; NMR (DMSO-d₆) 7.25-7.11 (m, 5H), 7.08 (d, J = 8.2Hz, 2H), 6.68 (d, J = 8.6Hz, 2H), 4.14 (s, 1H), 4.09 (d, J = 9.2Hz, 1H), 3.33 (s, 2H), 3.30 (s, 1H), 2.74 (m, 1H--partially under water peak from the NMR solvent), 2.60-2.35 (m, 4H--partially under NMR solvent peak), 1.70-1.44 (m, 4H), 0.63 (d, J = 6.7Hz, 3H). Deuterium oxide washed out the singlets at 4.14 and 3.30 ppm.

This product was recrystallized from ethyl acetate to give purified title product; mp 213-214°C; IR (KBr) 3263, 3023, 2940, 2917, 1615, 1517, 1453, 1273, 1221, 1186, 1020, 1011, 831, 687. Anal. C 73.73, H 8.03, N 4.01, calcd. C 73.87, H 7.97, N 4.10.

### EXAMPLE 4

### 2-(4-Benzyl-4-hydroxypiperidino)-1-(4-(triisopropylsilyloxy)phenyl)-1-propanone

This product was prepared according to the procedures of Example A from 4-hydroxy-4-benzylpiperidine (2.72 g, 14.22 mmol), 4-triisopropylsilyloxyalpha-bromopropiophenone (5.48 g, 14.22 mmol), and triethylamine (2.0 ml, 14.35 mmol) in ethanol (50 ml) with a reflux time of 17 hours to give 4.92 g (70%) of present, chromatographed title product as an orange oil; NMR 8.01 (d, J = 8.8Hz, 2H), 7.31-7.22 (m, 3H), 7.18-7.15 (m, 2H), 6.87 (d, J=8.8Hz, 2H), 4.03 (q, J = 6.7Hz, 1H), 2.72 (s, 2H), 2.68-2.57 (m, 3H), 2.45 (dt, 1H), 1.78-1.42 (m, 4H), 1.40-1.25 (m, 7H), 1.10 (d, J = 7Hz, 18H).

### EXAMPLE 5

### (1S*,2S*)- and (1 R*,2S*)-2-(4-Benzyl-4-hydroxypiperidino)-1-(4-(triisopropylsilyloxy)phenyl)-1-propanol

Title product of the preceding Example (4.92 g, 9.33 mmol) was dissolved in ethanol (100 ml) and NaBH₄ (0.38 g, 10 mmol) was added all at once. After stirring overnight at ambient temperature, present (1S*,2S*)-title product was recovered by filtration, 2.11 g; NMR 7.46-7.17 (m, 7H), 6.84 (d, J = 7Hz, 2H), 4.18 (d, J = 11Hz, 1H), 2.86 (br t, 1H), 2.77 (s, 2H), 2.70-2.42 (m, 4H), 1.89-1.55 (m, 6H), 1.30-1.13 (m, 3H), 1.10 (d, J = 8.6 Hz, 18H), 0.75 (d, J = 6Hz, 3H).

The filtrate was concentrated and the residue dissolved in ethyl acetate, extracted with water (2x) and brine, dried (CaSO₄), concentrated to 2.33 g of a light yellow solid, and flash chromatographed on silica gel (2 x 6 inches, ethyl acetate/hexane gradient elution) to give first 1.4 g more of (1S*,2S*)-product, followed by 0.46 g of (1R*,2S*)-product; NMR 7.33-7.11 (m, 7H), 6.82 (d, J = 8.6Hz, 2H), 4.77 (d, J = 4Hz, 1H), 2.80-2.39 (m, 5H), 1.88-1.43 (m, 6H), 1.31-1.13 (m, 8H), 1.08 (d, J = 6. 7Hz , 18H), 0.84 (d, J = 6.9Hz, 3H).

### EXAMPLE 6

### (1R*,2S*)-2-(4-Benzyl-4-hydroxypiperidino)-1-(4-hydroxyphenyl)-1-propanol

By the method of Example C, using ethyl acetate/hexane gradient elution in chromatography, (1R*,2S*)-title product of the preceding Example (0.46 g, 0.92 mmol) was converted to 0.24 g (74%) of present title product as a monohydrate; mp 173-174°C; NMR (DMSO d₆ with D₂O added) 7.21-7.12 (m, 5R), 7.04 (d, J = 7.6Hz, 2H), 6.64 (d, J= 8.6Hz, 2H), 4.64 (d, J= 8.6Hz, 1H), 2.59 (s, 2H), 2.59-2.49 (m, 5H--partially under NMR solvent) 1.50-1.31 (m, 4H), 0.82 (d, J = 7Hz, 3H). Anal. C 70.26, H 7.96, N 3.85; calcd. for monohydrate, C 70.17, H 8.13, N 3.90.

### EXAMPLE 7

### 2-(4-Benzyl-4-hydroxypiperidino)-1-(4-fluoro-phenyl)-1-propanone

Title product, prepared as in Example 1 in 80% yield, was recrystallized from ether, mp 119.5-120°C; Anal. C 73.41, H 7.08, N 4.03, calcd. C 73.87, H 7.09, N 4.10.

### EXAMPLE 8

### (1S*,2S*)- and (1R*,2S*)-2-(4-Benzyl-4-hydroxypiperidino)-1-(4-fluorophenyl)-1-propanol

Title products, prepared as in Example 5, were separated by flash chromatography on silica gel (ethyl acetate-hexane then methanol-ethyl acetate gradient). The 1S*,2S* product eluted first in 84% yield as a solid which was recrystallized from ethanol/ether, mp 153.5-154.5°C. Anal. C 73.53, H 7.67, N 4.08, calcd. C 73.44, H 7.63, N 4.08.

The 1R*,2S* product, eluted second in 15% yield, was recrystallized from ethanol/ether, mp 145-146°C; Anal. C 73.18, H 7.59, N 4.06, calcd. C 73.44, H 7.63, N 4.08.

### EXAMPLE 9

### 2-(4-Benzyl-4-hydroxypiperidino)-1-(4-chlorophenyl)-1-propanone

Title product, prepared from 4-chloro-alpha-bromopropiophenone, following the procedure of Example 1 in 72% yield, was purified by flash chromatography on silica gel and recrystallized from ether, mp 135.5-136°C; Anal. C 70.11, H 6.70, N 3.85, calcd. C 70.48, H 6.76, N 3.91.

### EXAMPLE 10

### (1S*,2S*)- and (1R*,2S*)-2-(4-Benzyl-4-hydroxypiperidino)-1-(4-chlorophenyl)-1-propanol

Title products, prepared following the procedure of Example 5, were separated by the same chromatographic procedure. The 1S*,2S* product was obtained in 70% yield, mp 159.5-160.5°C (ethanol/ether); Anal. C 70.13, H 7.50, N 3.91, calcd. C 70.08, H 7.28, N 3.89.

The 1R*,2S* product was obtained in 7% yield, mp 150.5-151.5°C (ethanol/ether); Anal. C 69.62, H 7.38, N 3.92, calcd. C 70.08, H 7.28, N 3.89.

### EXAMPLE 11

### 2-(4-Benzyl-4-hydroxypiperidino)-1-(4-chlorophenyl)-1-ethanone

Title product was prepared following the procedure of Example 1 from 4-chloro-alpha-bromoacetophenone in 76% yield; NMR 7.93 (d, J = 8.5Hz, 2H), 7.39 (d, J = 8.6Hz, 2H), 7.31-7.16 (m, 5H), 3.74 (s, 2H), 2.74 (s, 2H), 2.73-2.71 (m, 2H), 2.43 (dt, J = 11.5, 2.4Hz, 2H), 1.80 (dt, J = 12.7, 4.3Hz, 2H), 1.50 (br d, J = 13.8Hz, 2H).

### EXAMPLE 12

### 2-(4-Benzyl-4-hydroxypiperidino)-1-(4-chlorophenyl)ethanol

Title product, prepared from 4-chloro-alpha-bromoacetophenone following the procedure of Example 5 in 83% yield, was recrystallized from ethanol/ether, mp 151-152°C; NMR 7.34-7.18 (m, 9H), 4.67 (dd, J = 10.5, 3.5 Hz, 1H), 4.18 (br s, 1H), 2.89-2.86 (m, 1H), 2.76 (s, 1H), 2.68-2.47 (m, 3H), 2.41-2.31 (m, 2H), 1.73 (dq, J = 13.3, 4.4Hz, 2H), 1.58-1.50 (m, 2H), 1.24 (s, 1H).

### EXAMPLE 13

### 2-(4-Benzyl-4-hydroxypiperidino)-1-(4-fluorophenyl)-1-ethanone

Title product was prepared following the procedure of Example 1 in 59% yield from 4-fluoro-alpha-bromoacetophenone; NMR 8.05-7.99 (m, 2H), 7.33-7.04 (m, 7H), 3.76 (s, 2H), 2.83-2.71 (m, 4H), 2.43 (dt, J =11.5, 2.1Hz, 2H), 1.82 (dt, J = 12.7, 4.3Hz, 3H), 1.51 (br d, J = 11.5Hz, 2H).

### EXAMPLE 14

### 2-(4-Benzyl-4-hydroxypiperidino)-1-(4-fluorophenyl)-1-ethanol

Title product, prepared following the procedure of Example 5 in 85% yield, was recrystallized from ethanol/ether, mp 144.5-146°C; NMR 7.35-7.25 (m, 5H), 7.19 (d, J = 6.4Hz, 2H), 7.01 (t, J = 8.7Hz, 2H), 4.67 (dd, J = 10.5, 3.5Hz, 1H), 4.18 (br s, 1H), 2.88 (br d, J = 11.2Hz, 1H), 2.76 (s, 2H), 2.68-2.31 (m, 5H), 1.81-1.66 (m, 2H), 1.58-1.50 (m, 2H), 1.28 (s, 1H).

### EXAMPLE 15

### 1-(4-(Triisopropylsilyloxy)phenyl)-2-(4-hydroxy-4-phenylpiperidino)-1-propanone

By the procedure of Example A, 4-hydroxy-4-phenylpiperidine was converted to present title product in 37% yield as a clear oil; NMR 8.03 (d, J = 8.5Hz, 2H), 7.47 (d, J = 8Hz, 2H), 7.33 (t, J = 7.5Hz, 2H), 7.26-7.24 (m, 1H), 6.89 (d, J = 8.5Hz, 2H), 4.08 (q, J = 7.5Hz, 1H), 2.90-2.60 (m, 2H), 2.25-2.10 (m, 2H), 1.85-1.75 (m, 2H), 1.65-1.55 (m, 2H), 1.32-1.22 (m, 6H), 1.10 (d, J = 7Hz, 18H).

### EXAMPLE 16

### (1S*,2S*)-1-(4-(Triisopropylsilyloxy)phenyl)-2-(4-hydroxy-4-phenylpiperidino)-1-propanol

By the procedure of Example 5, title product of the preceding Example was converted to present title product in 87% yield; mp 148-151°C; NMR 7.52 (d, J = 7Hz, 2H), 7.38 (t, J = 7Hz, 2H), 7.30-7.25 (m, 1H), 7.19 (d, J = 8.5Hz, 2H), 6.84 (d, J = 8.5Hz, 2H), 4.23 (d, J = 9.5Hz, 1H), 3.13-3.02 (m, 1H), 2.80-2.58 (m, 3H), 2.30-2.08 (m, 2H), 1.90-1.78 (m, 2H), 1.29-1.17 (m, 3H), 1.09 (d, J = 7Hz, 18H), 0.79 (d, J = 6.5Hz, 3H).

### EXAMPLE 17

### (1S*,2S*)-1-(4-Hydroxyphenyl)-2-(4-hydroxy-4-phenylpiperidino)-1-propanol

Title product, prepared following the procedure of Example C from title product of the preceding Example in 65% yield, was recrystallized from ethanol; mp 202-204°C; Anal. C 71.95, H 8.09, N 4.26, calcd. for 0.5 C₂H₅OH, C 71.97, H 8.05, N 4.00.

### EXAMPLES 18-48

Using the methods of the preceding Examples, the following additional compounds were prepared, specifying yield to purified material in the final step:
18. 2-(4-Benzyl-4-hydroxypiperidino)-1-(4-hydroxyphenyl)ethanol; 42%; mp 98-99°C (from ethanol).
19. (1S*,2S*)-2-(4-Benzyl-4-hydroxypiperidino)-1-(4-methoxyphenyl)-1-propanol; 36%; mp 145.5-146°C (from ethanol/ether),
20. (1S*,2S*)-2-(4-Benzyl-4-hydroxypiperidino)-1-(4-hydroxyphenyl)-1-pentanol; 55%; mp 158-159°C (from ethanol/ether).
21. (1R*,2S*)-2-(4-Benzyl-4-hydroxpiperidino)-1-(4-hydroxyphenyl)-1-pentanol; 37%; mp 156-157°C (from ether).
22. (1S*,2S*)-2-(4-Benzyl-4-hydroxypiperidino)-1-(4-hydroxyphenyl)-1-butanol; 53%; mp 190-191°C (from ethanol).
23. (1S*,2S*)-2-(4-Benzyl-4-hydroxypiperidino)-1-(4-methoxyphenyl)-1-butanol; 61%; mp 143-144°C (purified by flash chromatography on silica gel using ethyl acetate/hexane gradient elution).
24. 2-(4-Benzyl-4-hydroxypiperidino)-1-(4-cyanophenyl)ethanol; 52%; mp 142-143°C (from ethanol/ether/ hexane).
25. 2-(4-Benzyl-4-hydoxypiperidino)-1-(2-hydroxyphenyl)ethanol; 43%; mp 172-173.5°C (from ethanol).
26. 2-(4-Benzyl-3-hydroxypiperidino)-1-(3-hydroxyphenyl)ethanol; 76%; mp 198-199°C (from ethanol).
27. 1-(4-Chlorophenyl)-2-(4-hydroxy-4-phenylpiperidino)ethanol; 63%; mp 155.5-157°C (from ethanol/ether).
28. (1S*,2S*)-2-(4-(4-chlorophenyl)-4-hydroxypiperidino)-1-(4-hydroxyphenyl)-1-propanol; 58%; mp 204-206°C (from ethyl acetate).
29. 2- (4-Hydroxy-4-phenylpiperidino)-1-(2-thienyl)ethanol; 54%; mp 167-168°C (from ethanol).
30 . 1-(4-Trifluoromethylphenyl)-2-(4-hydroxy-4-phenylpiperidino)ethanol; 34%; mp 152-153°C (from ethanol/ether).
31. 1-(4-Acetamidophenyl)-2-(4-hydroxy-4-phenylpiperidino)ethanol; 34%; mp 217.5-218°C (from ethanol).
32. (1S*,2S*)-1-(4-hydroxyphenyl)-2-[4-hydroxy-4-(2-phenylethyl)piperidino]-1-propanol; 51%; mp 200-201°C (from ethyl acetate).
33 . (1S*,2S*)-1-(4-Hydroxyphenyl)-2-[4-hydroxy-4-(3-phenylpropyl)piperidino]-1-propanol; 46%; mp 200.5-201°C (from ethyl acetate).
34. (1S*,2S*)-2-[4-(4-Fluorophenyl)-4-hydroxypiperidino]-1-(4-hydroxyphenyl)-1-propanol; 37%; mp 197-198°C (from ethyl acetate).
35. 1-(4-Cyanophenyl)-2-(4-hydroxy-4-phenylpiperidino)ethanol; 51%; mp 140-140.5°C (from ethanol/ether).
36. (1S*,2S*)-1-(4-Hydroxyphenyl)-2-[4-hydroxy-4-(4-methylphenyl)piperidino]-1-propanol; 41%; mp 188-189°C.
37. 1-(4-Carbamoylphenyl)-2-(4-hydroxy-4-phenylpiperidino)ethanol; 23% mp 213.5-215°C (from ethanol).
38. (1S*, 2S*)-1-(4-Fluorophenyl)-2-(4-hydroxy-4-phenylpiperidino)-1-propanol; 44%; mp 177-179°C (from ethanol).
39. (1R*,2S*)-1-(4-Hydroxyphenyl)-2-(4-hydroxy-4-phenylpiperidino)-1-propanol; 25%; mp 152-155°C (from ethanol).
40. (1S*,2S*)-2-(4-Hydroxy-4-phenylpiperidino)-1-phenyl-1-propanol; 50%; mp 149-152°C (from ethyl acetate/hexane).
41. (1S*,2S*)-1-(4-chlorophenyl)-2-(4-hydroxy-4-phenylpiperidino)-1-propanol; 38%; mp 192-194°C (from ethanol).
42. 1-(4-Aminophenyl)-2-(4-hydroxy-4-phenylpiperidino)ethanol; 47%; mp 156.5-158°C (from ethyl acetate/ether).
43. (1S*,2S*)-2-(3-Benzyl-3-hydroxypyrrolidino)-1-(4-hydroxyphenyl)-1-propanol; 71%; mp 134-136°C.
44. (1S*,2S*)-1-(4-Hydroxyphenyl)-2-(3-hydroxy-3-phenylpyrrolidino)-1-propanol; 56%; mp 74-78°C.
45. (1S*,2S*)-1-(4-Chlorophenyl)-2-[4-hydroxy-4-(2-phenylethyl)piperidino]-1-propanol; 33%; mp 152-154°C (from ethanol).
46. (1S*,2S*)-1-(4-Hydroxyphenyl)-2-[4-hydroxy-4-(4-Phenylbutyl)piperidino]-1-propanol; 44%; mp 191-192°C (from ethanol).
47. 1-(4-Carboxyphenyl)-2-(4-hydroxy-4-phenylpiperidino)ethanol; 98%; mp 254.5-255°C (from H₂O).
48. 2-(4-Hydroxy-4-phenylpiperidino)-1-[4-(methoxycarbonyl)phenyl]ethanol; 57%; mp 138.5-139.5°C (from ethanol/ether).

### EXAMPLE 49

### (R-1-(4-Chlorophenyl)-2-(4-hydroxy-4-phenylpiperidino)ethanol

4-Hydroxy-4-phenylpiperidine (177 mg, 1 mmol) was dissolved in dry tetrahydrofuran (13 mL) and chilled to -15°C with stirring and under a nitrogen atmosphere. Butyllithium (0.8 ml, 2 mmol, 2.5N) was added dropwise over 3 minutes. (-)-R-1-(4-chlorophenyl)ethylene oxide [155 mg, 1 mmol; J. Am. Chem. Soc. 109, 7925 (1987); J. Org. Chem. 53, 2861 (1988)] was dissolved in 1 mL tetrahydrofuran and added to the cold reaction with a 1 mL rinse. The mixture was warmed to ambient temperature and finally refluxed overnight. The mixture was cooled to room temperature and quenched with solid NaHCO₃. The crude reaction was directly chromatographed on silica gel using on ethyl acetate-hexane gradient elution. The product containing fractions were carefully rechromatographed on silica gel with 50% ethyl acetate-hexane elution to give 112 mg (33%) of product as an oily foam. Trituration with ether-hexane gave 15.2 mg of cream colored product which had mp 130-113°C; [alpha]_{D} = -18°.

(S)-1-(4-(Chlorophenyl)-2-(4-hydroxy-4-phenylpiperidino)ethanol, having the same physical properties except for sign of rotation was prepared in the same manner from (+)-S-1-(4-chlorophenyl)ethylene oxide.

### EXAMPLE 50

### Enantiomeric (1S,2S)- and (1R,2R)-1-(4-Hydroxyphenyl)-2-(4-hydroxy-4-phenylpiperidino)-1 -propanols

(+)-Tartaric acid (300 mg, 2 mmol) was dissolved in 30 mL warm methanol. Racemic 1S*,2S*-1-(4-hydroxyphenyl)-2-(4-hydroxy-4-phenylpiperidinyl)propanol (655 mg, 2 mmol) was added all at once. With stirring and gentle warming a colorless homogeneous solution was obtained. Upon standing at ambient temperature 24 hours, 319 mg (66%) of a fluffy white precipitate was obtained. This product was recrystallized from methanol to give 263 mg of the (+)-tartrate salt of dextrorotatory title product as a white solid; mp 206.5-207.5°C; [alpha]_{D} = -36.2°C. This salt (115 mg) was added to 50 ml of saturated NaHCO₃. Ethyl acetate (5 ml) and the mixture was vigorously stirred 30 minutes. The aqueous phase was repeatedly extracted with ethyl acetate. The organic layers were combined and washed with brine, dried over calcium sulfate, and concentrated. The tan residue was recrystallized from ethyl acetate-hexane to give 32 mg (39%) of white, dextrorotatory title product; mp 203-204°C; [alpha]_{D} = +56.9°. Anal. Calcd. for C₂₀H₂₅NO₃: C, 73.37; H, 7.70; N, 4.28. Found: C, 72.61; H, 7.45; N, 4.21.

The filtrate from the (+)-tartrate salt preparation above was treated with 100 ml saturated aqueous NaHCO₃ and extracted well with ethyl acetate. The combined organic extracts were washed with brine, dried over calcium sulfate and concentrated to give 380 mg of recovered starting material (partially resolved). This material was treated with (-)-tartaric acid (174 mg) in 30 mL of methanol as above. After standing for 24 hours, filtration gave 320 mg (66%) of product which was further recrystallized from methanol to produce 239 mg the (-)-tartrate salt of levorotatory title product; mp 206.5-207.5°C; [alpha]_{D} = +33.9°. The latter was converted to levorotatory title product in the manner above in 49% yield; mp 204-205°C; [alpha]_{D} = -58.4°. Anal. Found: C, 72.94; H, 7.64; N, 4.24.

## Claims

1. A compound of the formula 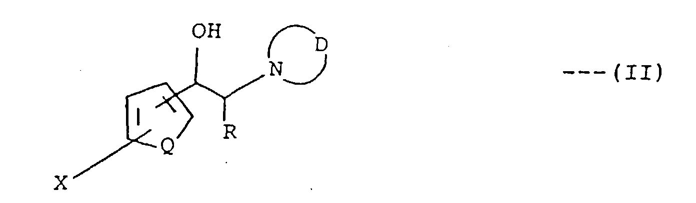 wherein
D is 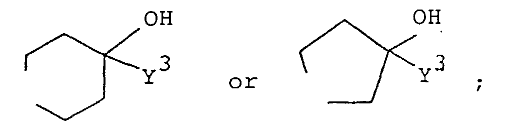
R is H, (C₁-C₆)alkyl, (C₂-C₆)alkenyl or (C₂-C₆)alkynyl;
Q is S or CH=CH;
X is hydrogen, (C₁-C₃)alkyl, halo, OR¹, OCOR¹, CO₂R¹, SR¹, NHR¹, NHCOR¹, CONH₂ or CN;
R¹ is hydrogen or (C₁-C₃)alkyl;
Y³ is 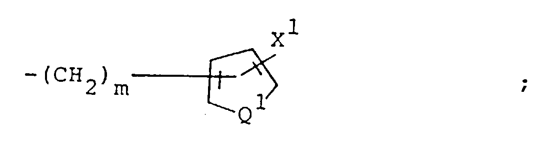
Q¹ is independently a value of Q as defined above;
X¹ is independently a value of X as defined above; and
m is 0, 1, 2, 3 or 4; or
a pharmaceutically-acceptable acid addition salt thereof.

2. A compound of claim 1 wherein Q is CH=CH, X is substituted at the 4-position of the phenyl ring and is hydroxy, fluoro or chloro.

3. A compound of claim 2 wherein R is hydrogen, X is 4-hydroxy and Y³ is benzyl, 2-phenylethyl or 3-phenylpropyl.

4. A compound of claim 3 wherein R is methyl having 1S*,2S* relative stereochemistry:

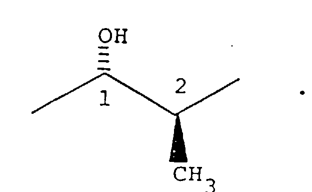

5. A compound of the formula 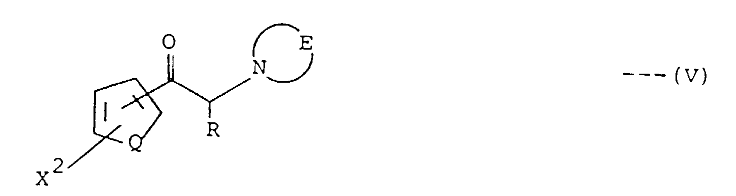 wherein
E is 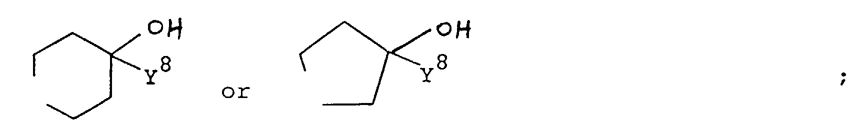
R is hydrogen, (C₁-C₆)alkyl, (C₂-C₆)alkenyl or (C₂-C₆)alkynyl;
Q is S or CH=CH;
X² is hydrogen, (C₁-C₃)alkyl, halo, OR¹, OR², COOR¹, OCOR¹, SR¹, SR², NHR¹, NR¹R³, NHCOR¹, CONH₂ or CN;
R¹ is hydrogen or (C₂-C₃)alkyl;
R² is a conventional hydroxy or mercaptan protecting group;
R³ is a conventional amino protecting group;
Y⁸ is 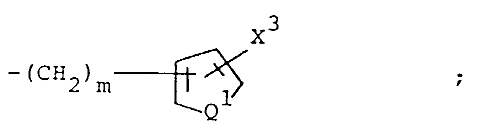
Q¹ is independently a value of Q as defined above;
x³ is independently a value of X² as defined above; and
m is 0, 1, 2, 3 or 4;

6. The compound according to claim 1 wherein said compound is (+)-(1S,2S)-1-(4-hydroxyphenyl])-2-(4-hydroxy-4-phenylpiperidinyl)-1-propanol.

7. The compound according to claim 1 said compound is (-)-(1R,2R)-1-(4-hydroxyphenyl)-2-(4-hydroxy-4-phenylpiperidinyl)-1-propanol.

8. An antiischemic composition comprising a compound of the formula II as claimed in any one of claims 1 to 4, or a pharmaceutically acceptable salt thereof, together with a diluent or carrier.

9. A compound of the formula II or a pharmaceutically acceptable salt thereof as claimed in any one of claims 1 to 4 for use as a medicament.

10. The use of a compound of the formula II as claimed in any one of claims 1 to 4, or a pharmaceutically acceptable salt thereof, for the manufacture of a medicament for treating degenerative central nervous system disorders such as stroke, Alzheimer's disease, Parkinson's disease and Huntington's disease in an animal.

## Patentansprüche

1. Verbindung der Formel 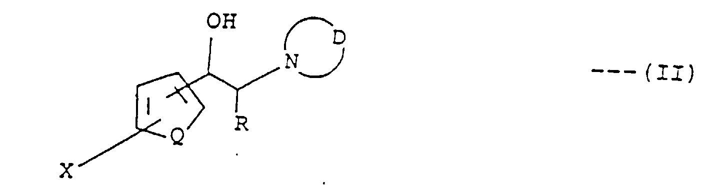
worin D 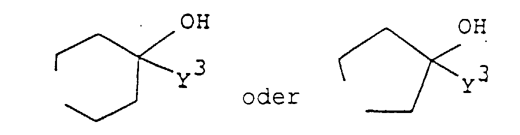 ist;
R H, ein (C₁-C₆)-Alkyl-, (C₂-C₆)-Alkenyl- oder (C₂-C₆)-Alkinylrest ist;
Q S oder CH=CH ist;
X Wasserstoff, ein (C₁-C₃)-Alkylrest, Halogen, OR¹, OCOR¹, CO₂R¹, SR¹, NHR¹, NHCOR¹, CONH₂ oder CN ist;
R¹ Wasserstoff oder ein (C₁-C₃)-Alkylrest ist,
Y³ 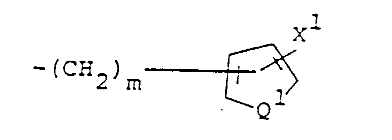 ist,
Q¹ unabhängig eine Bedeutung hat, wie für Q oben definiert;
X¹ unabhängig eine bedeutung hat, wie für X oben definiert und
m 0, 1, 2, 3 oder 4 ist oder ein pharmazeutisch annehmbares Säureadditionssalz davon.

2. Verbindung nach Anspruch 1, worin Q CH=CH ist, X an Position 4 des Phenylrings substituiert ist und ein Hydroxy-, Fluor- oder Chlorrest ist.

3. Verbindung nach Anspruch 2, worin R Wasserstoff ist, X ein 4-Hydroxyrest ist und Y³ ein Benzyl-, 2-Phenylethyl-oder 3-Phenylpropylrest ist.

4. Verbindung nach Anspruch 3, worin R ein Methylrest ist mit der relativen 1S*,2S*-Stereochemie:

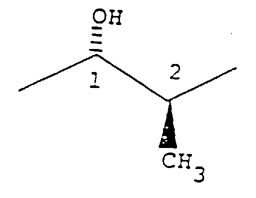

5. Verbindung der Formel 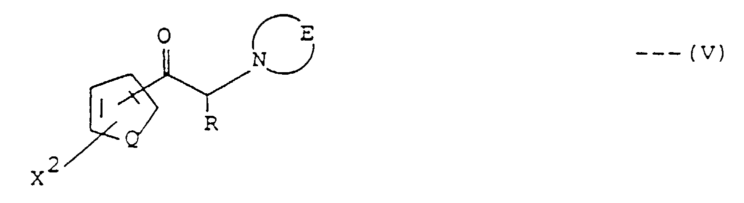
worin E 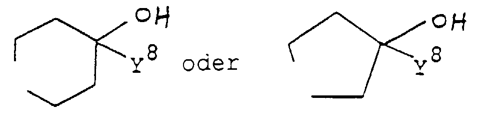 ist,
R Wasserstoff, ein (C₁-C₆)-Alkyl-, (C₂-C₆)-Alkenyl- oder (C₂-C₆)-Alkinylrest ist;
Q S oder CH=CH ist;
X² Wasserstoff, ein (C₁-C₃)-Alkylrest, Halogen, OR¹, OR², COOR¹, OCOR¹, SR¹, SR², NHR¹, NR¹R³, NHCOR¹, CONH₂ oder CN ist;
R¹ Wasserstoff oder ein (C₂-C₃)-Alkylrest ist;
R² eine übliche Hydroxy- oder Mercaptan-Schutzgruppe ist;
R³ eine übliche Amino-Schutzgruppe ist;
Y⁸ 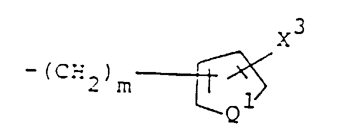 ist,
Q¹ unabhängig eine Bedeutung hat, wie für Q oben definiert,
X³ eine Bedeutung hat, wie für X² oben definiert und
m 0, 1, 2, 3 oder 4 ist.

6. Verbindung nach Anspruch 1, worin die Verbindung (+)-(1S,2S)-1-(4-Hydroxyphenyl)-2-(4-hydroxy-4-phenylpiperidinyl)-1-propanol ist.

7. Verbindung nach Anspruch 1, worin die Verbindung (-)-(1R,2R)-1-(4-Hydroxyphenyl)-2-(4-hydroxy-4-phenylpiperidinyl)-1-propanol ist.

8. Antiischämische Zusammensetzung umfassend eine Verbindung der Formel II, wie in einem der Ansprüche 1 bis 4 beansprucht, oder ein pharmazeutisch annehmbares Salz davon zusammen mit einem Verdünnungsmittel oder Träger.

9. Verbindung der Formel II oder ein pharmazeutisch annehmbares Salz davon nach einem der Ansprüche 1 bis 4 zur Verwendung als Arzneimittel.

10. Verwendung einer Verbindung der Formel II nach einem der Ansprüche 1 bis 4 oder eines pharmazeutisch annehmbaren Salzes davon zur Herstellung eines Arzneimittels zur Behandlung von degenerativen Störungen des Zentralnervensystems, wie Schlaganfall, Alzheimer Krankheit, Parkinson-Krankheit und Chorea Huntington bei einem Tier.

## Revendications

1. Composé de formule 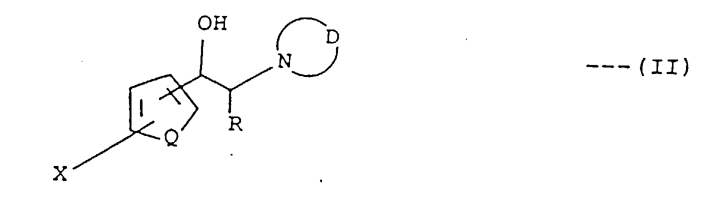 dans laquelle
D représente un groupe 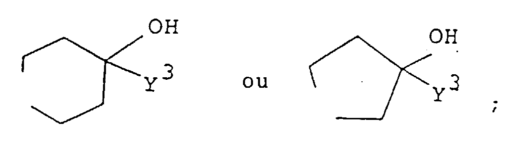
R représente H, un groupe alkyle en C₁ à C₆, alcényle en C₂ à C₆ ou alcynyle en C₂ à C₆ ;
Q représente S ou un groupe CH=CH ;
X représente l'hydrogène, un groupe alkyle en C₁ à C₃, halogéno, OR¹, OCOR¹, CO₂R¹, SR¹, NHR¹, NHCOR¹, CONH₂ ou CN ;
R¹ représente l'hydrogène ou un groupe alkyle en C₁ à C₃ ;
Y³ représente un groupe 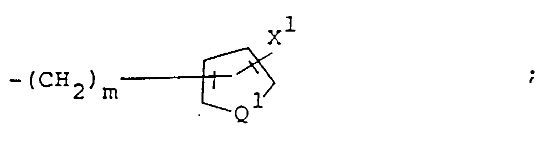
Q¹ a, indépendamment, une valeur de Q définie ci-dessus ;
X¹ a, indépendamment, une valeur de X définie ci-dessus ; et
m est égal à 0, 1, 2, 3 ou 4 ; ou
un de ses sels d'addition d'acides pharmaceutiquement acceptables.

2. Composé suivant la revendication 1, dans lequel Q représente un groupe CH=CH, X est présent comme substituant en position 4 du noyau phényle et représente un groupe hydroxy, fluoro ou chloro.

3. Composé suivant la revendication 2, dans lequel R représente l'hydrogène, X représente un groupe 4-hydroxy et Y³ représente un groupe benzyle, 2-phényléthyle ou 3-phénylpropyle.

4. Composé suivant la revendication 3, dans lequel R représente un groupe méthyle, ayant la stéréochimie relative 1S*,2S*

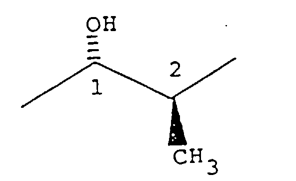

5. Composé de formule 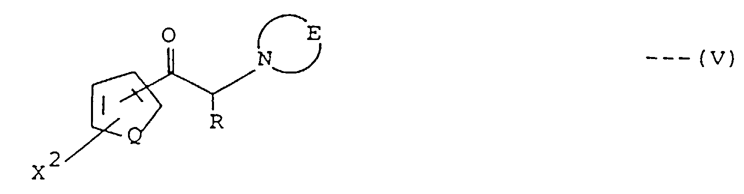 dans laquelle
E représente un groupe 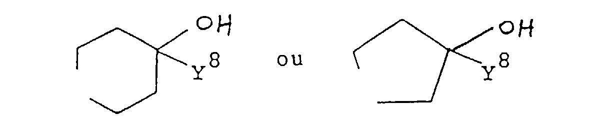
R représente l'hydrogène, un groupe alkyle en C₁ à C₆, alcényle en C₂ à c₆ ou alcynyle en C₂ à C₆ ;
Q représente S ou un groupe CH=CH ;
X² représente l'hydrogène, un groupe alkyle en C₁ à C₃, halogéno, OR¹, OR², COOR¹, OCOR¹, SR¹, SR², NHR¹, NR¹R³, NHCOR¹, CONH₂ ou CN ;
R¹ représente l'hydrogène ou un groupe alkyle en C₂ à C₃ ;
R² représente un groupe classique de protection de la fonction hydroxy ou mercaptan ;
R³ représente un groupe classique de protection de la fonction amino ;
Y⁸ représente un groupe 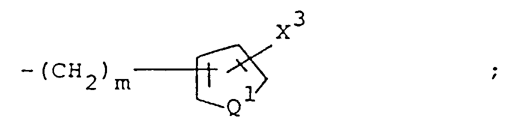
Q¹ a, indépendamment, une valeur de Q définie ci-dessus ;
X³ a, indépendamment, une valeur de X² définie ci-dessus ; et
m est égal à 0, 1, 2, 3 ou 4.

6. Composé suivant la revendication 1, qui consiste en le (+)-(1S,2S)-1-(4-hydroxyphényl)-2-(4-hydroxy-4-phénylpipéridinyl)-1-propanol.

7. Composé suivant la revendication 1, qui consiste en le (-)-(1R,2R)-1-(4-hydroxyphényl)-2-(4-hydroxy-4-phénylpipéridinyl)-1-propanol.

8. Composition anti-ischémique comprenant un composé de formule II suivant l'une quelconque des revendications 1 à 4, ou un de ses sels pharmaceutiquement acceptables, en association avec un diluant ou support.

9. Composé de formule II ou un de ses sels pharmaceutiquement acceptables suivant l'une quelconque des revendications 1 à 4, destiné à être utilisé comme médicament.

10. Utilisation d'un composé de formule II suivant l'une quelconque des revendications 1 à 4, ou d'un de ses sels pharmaceutiquement acceptables, pour la production d'un médicament destiné au traitement d'affections dégénératives du système nerveux central tel que l'ictus, la maladie d'Alzheimer, la maladie de Parkinson et la maladie de Huntington chez un animal.
